# EUROPEAN PATENT SPECIFICATION

(11) **EP 1 345 598 B1**
(45) Date of publication and mention of the grant of the patent: **05.09.2007**
(21) Application number: 01271216.2
(22) Date of filing: 12.12.2001
(51) Int. Cl.: A61K 31/427, A61P 25/18, A61P 25/24

(54) **The use of rosiglitazone for treating psychiatric disorders.**
Die Verwendung von Rosiglitazon zur Behandlung von psychiatrischen Erkrankungen.
L'utilisation de rosiglitazone pour le traitement de troubles psychiatriques.

(30) Priority: 18.12.2000 GB 0030845
(43) Date of publication of application: 24.09.2003
(73) Proprietor: SMITHKLINE BEECHAM PLC, Brentford, Middlesex TW8 9GS (GB)
(72) Inventor: CHAPMAN, Gayle, c/o GlaxoSmithKline, Harlow, Essex CM19 5AW (GB); VINSON, Mary, c/o GlaxoSmithKline, Harlow, Essex CM19 5AW (GB)
(74) Representative: Rutter, Keith
(86) International application number: PCT/GB2001/005488
(87) International publication number: WO 2002/049626

(56) References cited:
- WO-A-00/32190
- WO-A-00/35437
- WO-A-00/53601
- WO-A-00/62766
- US-A- 6 087 384
- PATENT ABSTRACTS OF JAPAN vol. 2000, no. 13, 5 February 2001 (2001-02-05) & JP 2000 273040 A (SANKYO CO LTD), 3 October 2000 (2000-10-03)
- COMBS, C.K., ET AL.: "Inflammatory mechanisms in Alzheimer's disease: inhibition of beta-amyloid-stimulated proinflammatory responses and neurotoxicity by PPAR-gamma agonists" JOURNAL OF NEUROSCIENCE, vol. 20, no. 2, 2000, pages 558-567, XP001064158
- SUNDARARAJAN, S. ET AL: "PPAR-gamma agonists reduce ischemic injury and immunoreactivity against inflammatory markers in rats." SOCIETY FOR NEUROSCIENCE ABSTRACTS, vol. 26, 2000, page 670.8 XP001064152

## Description

This invention relates to a novel treatment and in particular the treatment of psychiatric disorders.

Increased regeneration of damaged axons and enhanced synaptic plasticity may be beneficial in a number of psychiatric disorders where decreased synaptic plasticity is thought to play a role in the disease pathology.

Diseases where increased synaptic plasticity may be beneficial are the psychiatric disorders including schizophrenia and depression. It has been reported that patients undergoing chronic treatment with effective anti-depressants display increased markers of synaptic plasticity. Compounds that enhance the ability of neurons to extend neurites and potentially increase neuroplasticity may therefore be effective in the prophylaxis and treatment of these disorders.

European Patent Application, Publication Number 0306228 discloses certain thiazolidinedione derivatives which are disclosed *inter alia* as having hypoglycaemic and hypolipidaemic activity and activity in treating certain eating disorders. The compound of example 30 of EP 0306228 is 5-(4-[2-(N-methyl-N-(2-pyridyl)amino)ethoxy]benzyl)-2,4-thiazolidinedione (or'Compound (I)').

It is known that the γ-isoform of peroxisome proliferator-activated receptor (herein after PPARγ) is member of a nuclear receptor superfamily that includes receptors for the steroid, thyroid and retinoid hormones (Evans, Science 240, 889-895, (1988)).

It is known from J. Biol. Chem., 270,12963-12966 that thiazolidenediones such as Compound (I) are PPARγ agonists.

It is now surprisingly indicated that PPARγ agonists such as Compound (I) promote growth and/or repair of neurons and thus are indicated to be effective in the treatment and/or prophylaxis of diseases or conditions characterised by neuron degeneration, injury or impaired plasticity.

Accordingly, the invention provides the use of 5-(4-[2-(N-methyl-N-(2-pyridyl)amino)ethoxy]benzyl)-2,4-thiazolidinedione or a tautomeric form thereof, or a pharmaceutically acceptable salt thereof, or a pharmaceutically acceptable solvate thereof, for the manufacture of a medicament for the treatment of psychiatric disorders.

Diseases or conditions characterised by impaired plasticity include psychiatric disorders such as schizophrenia and depression; suitably schizophrenia; suitably depression.

Compound (I) may exist in one of several tautomeric forms, all of which are encompassed by the present invention as individual tautomeric forms or as mixtures thereof. Compound (I) also contains a chiral carbon atom, and hence exists in one or more stereoisomeric forms. It will be appreciated that the method of the present invention encompasses all of the said forms of Compound (I) whether as individual isomers or as mixtures of isomers, including racemates.

When used herein the term 'PPARγ agonist' relates to an agonist, such as a small molecular weight agonist, of the peroxisome proliferator-activated receptor of the gamma subtype, this nuclear receptor is a member of the ligand activated transcription factor family that include the steroid, retinoid and thyroid receptors.

PPARγ agonist activity may be assessed by use of the methodology disclosed by Lehmann et al : Journal of Biological Chem., 270, 12953-12956 (1995).

Suitable derivatives of Compound (1) are pharmaceutically acceptable derivatives, for example salts and solvates.

Suitable derivatives of Compound (I) include those disclosed in the above mentioned publications.

Suitable pharmaceutically acceptable salts include salts of salts derived from appropriate acids, such as acid addition salts, or bases.

Suitable pharmaceutically acceptable salts include metal salts, such as for example aluminium, alkali metal salts such as lithium, sodium or potassium, alkaline earth metal salts such as calcium or magnesium and ammonium or substituted ammonium salts, for example those with lower alkylamines such as triethylamine, hydroxy alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-b-phenethylamine, dehydroabietylamine, N,N'-bisdehydroabietylamine, glucamine, N-methylglucamine or bases of the pyridine type such as pyridine, collidine, quinine or quinoline.

Suitable acid addition salts include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methane-sulphonate, a-keto glutarate and a-glycerophosphate, especially the maleate salt.

Suitable pharmaceutically acceptable salts of Compound (I) are as disclosed in EP 0306228 and WO94/05659 and include maleate salts.

Suitable pharmaceutically acceptable solvates include hydrates.

Suitable pharmaceutically acceptable solvates of Compound (I) are as disclosed in EP 0306228 and WO94/05659 and include hydrates.

Compound (1), or the tautomeric form thereof, and/or a pharmaceutically acceptable salt thereof, and/or a pharmaceutically acceptable solvate thereof, may be prepared using the processes described in EP 0306228 and WO94/05659.

In the above mentioned treatment Compound (I) or a tautomeric form thereof, or a pharmaceutically acceptable salt thereof, or a pharmaceutically acceptable solvate thereof, may be administered per se or, preferably, as a pharmaceutical composition also comprising a pharmaceutically acceptable carrier.

In the treatment of the invention, Compound (I) or a tautomeric form thereof, or a pharmaceutically acceptable salt thereof, or a pharmaceutically acceptable solvate thereof is formulated and administered in accordance with the methods disclosed in the above mentioned patent applications and patents.

As used herein the term 'pharmaceutically acceptable' embraces compounds, compositions and ingredients for both human and veterinary use: for example the term 'pharmaceutically acceptable salt' embraces a veterinarily acceptable salt.

The active compounds are usually administered as the sole medicament agent but they may be administered in combination with other medicament agents.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Suitable dosages of Compound (I) include the known doses for these compounds as described or referred to in reference texts such as the British and US Pharmacopoeias, Remington's Pharmaceutical Sciences (Mack Publishing Co.), Martindale The Complete Drug Reference (London, The Pharmaceutical Press, 32nd Edition) or the above mentioned publications or doses which can be determined by standard procedures.

Suitable dosages of the Compound (I) include those disclosed in EP 0306228 and WO94/05659 and 1, 2, 3, 4, 5, 6, 7, 8, 9, 10, 11 or 12 mg of Compound (I).

Particular dosages of Compound (I) are 2mg, 4mg and 8mg.

The composition of the invention may be administered from 1 to 6 times a day, but most preferably 1 or 2 times per day, or some other such period as disclosed in the above mentioned publications.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

Compositions may, if desired, be in the form of a pack accompanied by written or printed instructions for use.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press, 32nd Edition) and Harry's Cosmeticology (Leonard Hill Books).

One index of synaptic plasticity is increased synaptic transmission. This can be measured in cultured hippocampal neurons using electrophysiological recordings as described by Levine E S, Crozier R A, Black I B, Plummer M R. "Brain derived neurotrophic factor modulates hippocampal synaptic transmission by increasing N-methyl-D aspartic acid receptor activity", in Proc. Natl. Acad. Sci USA Vol 95 pp10235-10239 (1998). Thus the neurons would be treated with test compound, such as Compound (I), and then their synaptic transmission determined against a control following glutamate exposure.

No adverse toxicological effects are expected for the compositions or methods of the invention in the above mentioned dosage ranges.

The following descriptions and examples illustrate the invention but do not limit it in any way.

### Description and Examples

### Primary neuronal cell culture

The hippocampi of gestational day 18 rat embryos were dissected out, incubated in trypsin (0.08%, 30min at 37 °C) and dissociated mechanically (Skaper et al., 19990). Hippocampal cells were resuspended in neurobasal medium supplemented with B27, anti-oxidants, 1mM glutamine, 25 µM glutamate, 1 mM pyruvate, +/- Compound 1 (10nM-5µM) or vehicle (DMSO) control.

For outgrowth assays, cells were plated at a density of 3000 cells/well into 96 well dishes that had previously been coated with poly-D-lysine followed by 10% FCS. For RNA isolation, cells were plated at 1x10⁶ cells/well into a 35mm tissue culture dish that had previously been coated with poly-D-lysine followed by 10% FCS.

### RNA isolation and reverse transcription

RNA was prepared from cells lysed in Tri- reagent (Sigma, Dorset, UK) using 1 ml of Tri-reagent per 10 cm² plate. Total RNA was extracted from the tissue according to the manufacturer's suggested protocol with the addition of an extra chloroform extraction step and phase separation and an extra wash of the isolated RNA in 70% ethanol. The RNA was resuspended in autoclaved, double distilled water and the concentration calculated by A₂₆₀ measurement. RNA quality was assessed by electrophoresis on a 1% agarose gel. First strand cDNA was synthesised using oligo(dT)₁₅ and 500ng of each RNA sample; 0.01 M dithiothreitol, 0.5 mM each dNTP, 0.5 µg oligo(dT)₁₅ primer, 40 U RNAseOUT ribonuclease inhibitor (Life Technologies, Paisley, UK), 200 U SuperscriptII reverse transcriptase (Life Technologies, Paisley, UK). Reverse transcription reactions were performed in duplicate along with an additional reaction in which the reverse transcriptase enzyme was omitted to allow for assessment of genomic DNA contamination of the RNA. Taqman PCR was carried out using an ABI prism 7700 sequence detector (Perkin Elmer, Cheshire, UK) under the following conditions; 50°C for 2 minutes, 95°C for 10 minutes followed by forty cycles of 95°C for 1 seconds, 60°C for 1 minute. The reaction mixture contained cDNA samples (5µl of 20 µl RT reaction); 2.5 mM MgCl₂, 0.2 mM dATP, dCTP, dGTP and dUTP, 0.1 µM each primer, 0.05 µM Taqman probe, 0.01 U AmpErase uracil-N-glycosylase (Perkin Elmer, Cheshire, UK), 0.0125 U Amplitaq Gold DNA polymerase (Perkin Elmer, Cheshire, UK). Taqman primer and probe set for rat PPAR-gamma were designed using Primer Express software (Perkin Elmer, Cheshire, UK). Primer and probe sequences (5' - 3') (forward primer, reverse primer, Taqman probe);
Forward primer: CTGACCCAATGGTTGCTGATTAC
Reverse primer: GGACGCAGGCTCTACTTTGATC
Probe: FAM-AAATATGACCTGAAGCTCCAAGAATACCAAAGTGC-TAMRA
(Amplicon is 80 bp and the amplicon coordinates within the rat PPARgamma mRNA (accession AB011365) are 176-255)

Triplicate amplifications were carried out, following amplification, a representitive amplicon from each sample (2µl) was electrophoressed on a 4% agarose gel to determine molecular weight.

### Neurite outgrowth assays

Compound (I) was solubilised in DMSO and added to culture medium at time of cell plating at a dilution of 1:1000. Vehicle only (1:1000) was added to culture medium of untreated controls. After 48 hours, cells were fixed with 4% paraformaldehyde for 1 hour on ice, washed with PBS and stained using Coomassie. Assays were quantified using a KS300 image analysis system (Imaging Associates, UK). For each cell measured, the length from the edge of the cell to the end of the longest neurite was measured for 100 cells/well for each treatment in triplicate. All data are means and SEM pooled from three independent experiments#. Results are expressed as a percentage of the length of neurites of cells treated with vehicle alone.
# Compound (I) at 0.01 and 0.05µM has been analysed in one experiment only (n=3)

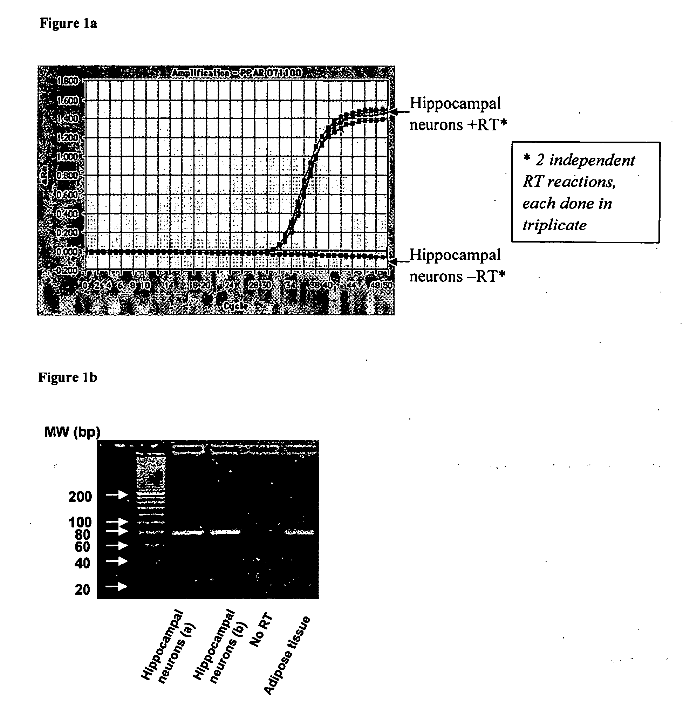

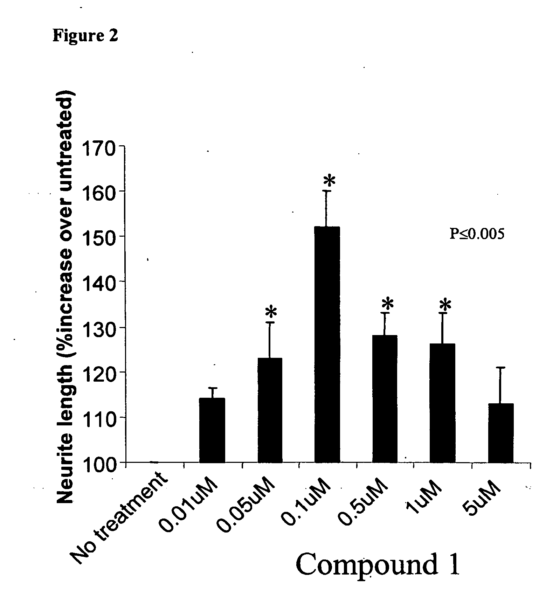

**Results:** the results are expressed in the attached Figures 1 (a), 1(b) and 2, which are as follows:
**Figure 1(a):** *PPAR-gamma is expressed by rat primary hippocampal neurons.* (a) Real-time PCR (Taqman) amplification plot of PPAR-gamma in cDNA derived from rat primary hippocampal neurons;
**Figure 1(b):** Amplicons from taqman analysis separated on 4% agarose gel. cDNA derived from adipose used as a positive control. Molecular weight as predicted; and
**Figure 2:** Compound (I) increases neurite outgrowth in rat primary hippocampal neurons in a dose dependant manner. Compound (I) at 100nM increases outgrowth over untreated controls by approx. 50%.

### References

Skaper SD, Facci L, Milani D, Leon A, and Toffano G (1990). In Methods in Neurosciences, Vol 2, Academic Press, San Diego.

## Claims

1. A use of S-(4-[2-(N-methyl-N-(2-pyridyl)amino)ethoxy]benzyl)-2,4-thiazolidinedione or a tautomeric form thereof, or a pharmaceutically acceptable salt thereof, or a pharmaceutically acceptable solvate thereof, for the manufacture of a medicament for the treatment of psychiatric disorders.

2. A use according to claim 1, wherein the pharmaceutically acceptable salt is a maleate salt.

3. A use according to claim 1 or claim 2, wherein the pharmaceutically acceptable solvate is a hydrate.

4. A use according to any one of claims 1 to 3, wherein the psychiatric disorders are selected from schizophrenia and depression.

5. A use according to any one of claims 1 to 4, wherein the psychiatric disorder is schizophrenia.

6. A use according to any one of claims 1 to 4, wherein the psychiatric disorder is depression.

## Patentansprüche

1. Verwendung von 5-(4-[2-(N-Methyl-N-(2-pyridyl)amino)ethoxy]benzyl)-2,4-thiazolidindion, oder einer tautomeren Form davon, oder eines pharmazeutisch verträglichen Salzes davon, oder eines pharmazeutisch verträglichen Solvats davon, zur Herstellung eines Medikaments zur Behandlung von psychiatrischen Erkrankungen.

2. Verwendung gemäß Anspruch 1, wobei das pharmazeutisch verträgliche Salz ein Maleatsalz ist.

3. Verwendung gemäß Anspruch 1 oder 2, wobei das pharmazeutisch verträgliche Solvat ein Hydrat ist.

4. Verwendung gemäß einem der Ansprüche 1 bis 3, wobei die psychiatrischen Erkrankungen ausgewählt sind aus Schizophrenie und Depression.

5. Verwendung gemäß einem der Ansprüche 1 bis 4, wobei die psychiatrische Erkrankung Schizophrenie ist.

6. Verwendung gemäß einem der Ansprüche 1 bis 4, wobei die psychiatrische Erkrankung Depression ist.

## Revendications

1. Utilisation de 5-(4-[2-(N-méthyl-N-(2-pyridinyl)-amino)éthoxy]benzyl)-2,4-thizolidinedione ou d'une de ses formes tautomères, ou d'un de ses sels pharmaceutiquement acceptables, ou d'un de ses produits de solvatation pharmaceutiquement acceptables, pour la production d'un médicament destiné au traitement de troubles psychiatriques.

2. Utilisation suivant la revendication 1, dans laquelle le sel pharmaceutiquement acceptable est un maléate.

3. Utilisation suivant la revendication 1 ou la revendication 2, dans laquelle le produit de solvatation pharmaceutiquement acceptable est un hydrate.

4. Utilisation suivant l'une quelconque des revendications 1 à 3, dans laquelle les troubles psychiatriques sont choisis entre la schizophrénie et la dépression.

5. Utilisation suivant l'une quelconque des revendications 1 à 4, dans laquelle le trouble psychiatrique est la schizophrénie.

6. Utilisation suivant l'une quelconque des revendications 1 à 4, dans laquelle le trouble psychiatrique est la dépression.
